# EUROPEAN PATENT APPLICATION

(11) **EP 4 438 585 A1**
(43) Date of publication of application: **02.10.2024**
(21) Application number: 22898597.4
(22) Date of filing: 24.11.2022
(51) Int. Cl.: C07C 29/34, C07C 31/125, C07B 61/00, B01J 23/80, B01J 29/072

(54) **METHOD FOR PRODUCING GUERBET ALCOHOL**

(30) Priority: 24.11.2021 JP 2021190480
(71) Applicant: Kao Corporation, Tokyo 103-8210 (JP)
(72) Inventor: ONOZAWA, Satoru, Wakayama-shi, Wakayama 640-8580 (JP)
(74) Representative: Hoffmann Eitle
(86) International application number: PCT/JP2022/043260
(87) International publication number: WO 2023/095814

(57) **Abstract**

A method of producing a Guerbet alcohol, including reacting a raw material alcohol having 8 or more and 36 or less carbon atoms, in the presence of a catalyst (A) containing a first component and a second component below, having a molar ratio of the first component with respect to the second component (first component/second component) of 2.9 or less, first component: copper, and second component: one kind selected from the group consisting of elements that are elements belonging to Groups 3 to 12 in the fourth to sixth periods of the periodic table, except copper and nickel.

## Description

### Field of the Invention

The present invention relates to a method of producing a Guerbet alcohol.

### Background of the Invention

It has been widely known that an aliphatic alcohol is reacted in the presence of a base catalyst or in the presence of a base catalyst and a cocatalyst to produce one molecule of a branched dimerized alcohol (i.e., a Guerbet alcohol) through removal of one molecule of water from two molecules of the alcohol, and the reaction has been referred to as Guerbet reaction.

In the case where a primary alcohol is used as the raw material alcohol as an example, it has been estimated that the reaction mechanism of the Guerbet reaction is constituted by the following elementary reactions (1) to (4):
(1) formation of an aldehyde through dehydrogenation of the alcohol,
(2) formation of an α,β-unsaturated aldehyde through aldol condensation of the aldehyde,
(3) formation of an allyl alcohol through reduction of the α,β-unsaturated aldehyde, and
(4) formation of a Guerbet alcohol through reduction of the allyl alcohol.

In the elementary reactions (1) to (4), various investigations have been reported for the kind and the amount of the base catalyst used, the kind and the amount of the cocatalyst used, and the like, for such purposes as the suppression of the side reaction, the enhancement of the reaction rate, the enhancement of the yield and the enhancement of the quality of the Guerbet alcohol formed, and the like.

For example, JP 2-286638 A (PTL 1) describes a method of producing a branched dimerized alcohol through reaction of an alcohol having 3 to 26 carbon atoms in the presence of (a) a catalyst formed of an alkaline substance and (b) a catalyst which contains copper, a fourth period transition metal element (e.g., nickel, chromium, cobalt, manganese, iron, and zinc), and a Group 8 platinum group element (e.g., platinum, palladium, ruthenium, and rhodium) in which the ratio copper/fourth period transition metal element is 1/9 to 9/1 (molar ratio), and the ratio Group 8 platinum group element/(copper + fourth period transition metal element) is 0.001 to 0.1 (molar ratio).

### Summary of the Invention

The present invention relates to a method of producing a Guerbet alcohol, including reacting a raw material alcohol having 8 or more and 36 or less carbon atoms, in the presence of a catalyst (A) containing a first component and a second component below, having a molar ratio of the first component with respect to the second component (first component/second component) of 2.9 or less.

### First component: copper

Second component: one kind selected from the group consisting of elements that are elements belonging to Groups 3 to 12 in the fourth to sixth periods of the periodic table, except copper and nickel

According to the method of producing a branched dimerized alcohol described in PTL 1, it is stated that the reaction time can be shortened, and the yield and the selectivity of the alcohol formed can be enhanced, as compared to the ordinary methods, but there is room for improvement from the standpoint of the shortening of the reaction time and the enhancement of the yield of the Guerbet alcohol formed. Furthermore, the hydrocarbon compound that is by-produced separately from the Guerbet alcohol formed is difficult to isolate in the purification, such as distillation, and there is a demand of decreasing the amount of the by-produced hydrocarbon compound from the standpoint of enhancing the purity of the Guerbet alcohol formed.

Under the circumstances, a problem to be solved by the present invention is to provide a method of producing a Guerbet alcohol, capable of shortening the reaction time, enhancing the yield of the Guerbet alcohol formed, and decreasing the amount of the by-produced hydrocarbon compound.

The present inventors have found that the problem can be solved by reacting a raw material alcohol having 8 or more and 36 or less carbon atoms in the presence of a particular catalyst (A).

The present invention relates to the following items [1] and [2].
[1] A method of producing a Guerbet alcohol, including reacting a raw material alcohol having 8 or more and 36 or less carbon atoms, in the presence of a catalyst (A) containing a first component and a second component below, having a molar ratio of the first component with respect to the second component (first component/second component) of 2.9 or less:
   first component: copper, and
   second component: one kind selected from the group consisting of elements that are elements belonging to Groups 3 to 12 in the fourth to sixth periods of the periodic table, except copper and nickel.
[2] A catalyst used for a method of producing a Guerbet alcohol, including a first component and a second component below, having a molar ratio of the first component with respect to the second component (first component/second component) of 2.9 or less:
   first component: copper, and
   second component: one kind selected from the group consisting of elements that are elements belonging to Groups 3 to 12 in the fourth to sixth periods of the periodic table, except copper and nickel.

The present invention can provide a method of producing a Guerbet alcohol, capable of shortening the reaction time, enhancing the yield of the Guerbet alcohol formed, and decreasing the amount of the by-produced hydrocarbon compound.

### Detailed Description of the Invention

The method of producing a Guerbet alcohol of the present invention includes reacting a raw material alcohol having 8 or more and 36 or less carbon atoms, in the presence of a catalyst (A) containing a first component and a second component below, having a molar ratio of the first component with respect to the second component (first component/second component) of 2.9 or less.

### First component: copper

Second component: one kind selected from the group consisting of elements that are elements belonging to Groups 3 to 12 in the fourth to sixth periods of the periodic table, except copper and nickel

The present invention exerts an effect of shortening the reaction time, enhancing the yield of the Guerbet alcohol formed, and decreasing the amount of the by-produced hydrocarbon compound. The mechanism therefor is not clear, but can be considered as follows.

As described above, in the case where a primary alcohol is used as the raw material alcohol as an example, it has been estimated that the reaction mechanism of the Guerbet reaction is constituted by the following elementary reactions (1) to (4):
(1) formation of an aldehyde through dehydrogenation of the alcohol,
(2) formation of an α,β-unsaturated aldehyde through aldol condensation of the aldehyde,
(3) formation of an allyl alcohol through reduction of the α,β-unsaturated aldehyde, and
(4) formation of a Guerbet alcohol through reduction of the allyl alcohol.

It is considered that the second component contribute to the microparticulation of the first component in the preparation process of the catalyst (A). It is considered that as a result, the surface area of the first component is increased through the microparticulation, and simultaneously the surface of the first component is activated, which accelerates the dehydrogenation reaction of the raw material alcohol on the surface of the first component in the elementary reaction (1) of the Guerbet reaction, resulting in a state where formed hydrogen is coordinated to the surface of the first component, or a state where hydrogen coordinated to the surface of the first component migrates to the surface of the second component and is coordinated to the surface of the second component. It is also considered that the molar ratio (first component/second component) of the first component and the second component contained in the catalyst (A) set to the particular value accelerates the reduction reaction of the α,β-unsaturated aldehyde on the surface of the second component having hydrogen coordinated thereto or on the surface of the first component having hydrogen coordinated thereto in the elementary reaction (3), and also accelerates the reduction reaction of the allyl alcohol on the surface of the second component having hydrogen coordinated thereto or on the surface of the first component having hydrogen coordinated thereto in the elementary reaction (4).

It is considered that in the elementary reaction (3) and the elementary reaction (4), the side reaction occurs to by-produce a hydrocarbon compound through the progress of the decarbonylation of the α,β-unsaturated aldehyde and the dehydration reaction and the reduction reaction of the allyl alcohol. It is considered that the catalyst (A) accelerates these elementary reactions to suppress the side reaction, and thereby the amount of the by-produced hydrocarbon compound is decreased.

In the following description, the decrease of the by-produced hydrocarbon compound may also be referred to as a low selectivity of the hydrocarbon compound.

The mechanism of the effects of the present invention exerted is not limited to the aforementioned concept.

### [Raw Material Alcohol]

In the method of producing a Guerbet alcohol of the present invention, an alcohol having 8 or more and 36 or less carbon atoms (which may be hereinafter referred simply to as a "raw material alcohol") is used.

The number of carbon atoms of the raw material alcohol is 8 or more, preferably 9 or more, and more preferably 10 or more, and is 36 or less, preferably 22 or less, more preferably 20 or less, and further preferably 18 or less, from the standpoint of the yield of the Guerbet alcohol and the low selectivity of the hydrocarbon compound.

One kind of the raw material alcohol may be used alone, or two or more kinds thereof may be used in combination.

Examples of the raw material alcohol include a primary aliphatic alcohol and a secondary aliphatic alcohol, among which a primary aliphatic alcohol is preferred, a primary aliphatic alcohol having 8 or more and 18 or less carbon atoms is more preferred, a saturated linear primary aliphatic alcohol having 8 or more and 18 or less carbon atoms is more preferred, a saturated linear primary aliphatic alcohol having 10 or more and 16 or less carbon atoms is further preferred, a saturated linear primary aliphatic alcohol having 10 or more and 14 or less carbon atoms is still further preferred, and a saturated linear primary aliphatic alcohol having 10 or more and 12 or less carbon atoms is still more further preferred, from the standpoint of the yield of the Guerbet alcohol and the low selectivity of the hydrocarbon compound.

Specific examples of the primary aliphatic alcohol include a saturated linear alcohol, such as 1-octanol (C8), 1-nonanol (C9), 1-decanol (C10), 1-undecanol (C11), 1-dodecanol (C12), 1-tridecanol (C13), 1-tetradecanol (C14), 1-pentadecanol (C15), 1-hexadecanol (C16), 1-heptadecanol (C17), 1-octadecanol (C18), 1-nonadecanol (C19), 1-eicosanol (C20), 1-heneicosanol (C21), and 1-docosanol (C22); a saturated alicyclic alcohol, such as cyclohexaneethanol (C8), cyclohexanepropanol (C9), and cyclohexanebutanol (C10); and an unsaturated alcohol, such as citronellol (C10) and oleyl alcohol (C18).

Specific examples of the secondary aliphatic alcohol include a saturated linear alcohol, such as 2-octanol (C8), 2-nonanol (C9), 2-decanol (C10), 2-undecanol (C11), 2-dodecanol (C12), 2-tridecanol (C13), 2-tetradecanol (C14), 2-pentadecanol (C15), 2-hexadecanol (C16), 2-heptadecanol (C17), 2-octadecanol (C18), 2-nonadecanol (C19), 2-eicosanol (C20), 2-heneicosanol (C21), and 2-docosanol (C22).

### [Catalyst (A)]

In the method of producing a Guerbet alcohol of the present invention, the catalyst (A) containing the particular components at the particular molar ratio is used, and the catalyst (A) that includes a carrier having the particular components supported thereon may also be used. The use of the catalyst (A) can shorten the reaction time, can enhance the yield of the Guerbet alcohol formed, and can decrease the amount of the by-produced hydrocarbon compound.

The catalyst (A) used in the present invention is a catalyst containing the first component and the second component shown below, and may also be a catalyst that includes a carrier having the first component and the second component shown below supported thereon.

### First component: copper

Second component: one kind selected from the group consisting of elements that are elements belonging to Groups 3 to 12 in the fourth to sixth periods of the periodic table, except copper and nickel.

### (First Component)

The first component of the catalyst (A) is not particularly limited, as far as the component is copper (Cu), and an oxide thereof may also be used.

The content of the first component (Cu) contained in the catalyst (A) is preferably 4% by mass or more, more preferably 6% by mass or more, further preferably 7% by mass or more, and still further preferably 10% by mass or more, from the standpoint of the yield of the Guerbet alcohol and the low selectivity of the hydrocarbon compound, and is preferably 55% by mass or less, more preferably 50% by mass or less, further preferably 45% by mass or less, and still further preferably 40% by mass or less, from the standpoint of the yield of the Guerbet alcohol and the economic efficiency.

The content of the first component contained in the catalyst (A) can be obtained specifically through measurement in the manner described in the examples.

The average primary particle diameter of the first component (Cu) contained in the catalyst (A) is preferably 0.2 nm or more, more preferably 1 nm or more, further preferably 3 nm or more, and still further preferably 10 nm or more, and is preferably 50 nm or less, more preferably 40 nm or less, and further preferably 30 nm or less, from the standpoint of the yield of the Guerbet alcohol and the low selectivity of the hydrocarbon compound.

The average primary particle diameter of the first component (Cu) contained in the catalyst (A) can be measured by the pulse method with a catalyst analyzer (product name: BELCAT-B, available from Nippon Bell Co., Ltd.).

### (Second Component)

The second component of the catalyst (A) is not particularly limited, as far as the component is one kind selected from the group consisting of elements that are elements belonging to Groups 3 to 12 in the fourth to sixth periods of the periodic table, except copper and nickel, and an oxide thereof may also be used.

In the second component, an element belonging to the fourth period of the periodic table is preferred, and zinc (Zn) is more preferred.

The content of the second component contained in the catalyst (A) is preferably 1% by mass or more, more preferably 3% by mass or more, further preferably 5% by mass or more, still further preferably 10% by mass or more, and still more further preferably 15% by mass or more, and is preferably 80% by mass or less, more preferably 75% by mass or less, further preferably 70% by mass or less, still further preferably 65% by mass or less, still more further preferably 50% by mass or less, even further preferably 45% by mass or less, and even still further preferably 40% by mass or less, from the standpoint of the yield of the Guerbet alcohol and the low selectivity of the hydrocarbon compound.

The content of the second component contained in the catalyst (A) can be obtained specifically through measurement in the manner described in the examples.

### (Third Component)

The catalyst (A) of the present invention may contain a third component other than the first component and the second component in such a range that does not impair the effects of the present invention.

Examples of the third component of the catalyst (A) include at least one kind selected from the group consisting of elements that are elements belonging to Groups 3 to 13 in the second to sixth periods of the periodic table, except copper, nickel, and the second component.

The content of the third component contained in the catalyst (A) is preferably 10% by mass or less, more preferably 5% by mass or less, further preferably 3% by mass or less, still further preferably 1% by mass or less, still more further preferably 0.6% by mass or less, even further preferably 0.3% by mass or less, even still further preferably 0.1% by mass or less, even still more further preferably 0.01% by mass or less, and yet further preferably 0% by mass.

The content of the third component contained in the catalyst (A) can be obtained through measurement in the same manner as in the first component and second component described above.

### (Carrier)

The catalyst (A) is preferably a catalyst including a carrier having the first component and the second component supported thereon from the standpoint of the productivity.

The carrier of the catalyst (A) is not particularly limited, as far as the carrier can support the first component and the second component.

Examples of the carrier of the catalyst (A) include a carbon material, such as activated carbon, nano carbon, and carbon black; and an inorganic material, such as aluminum oxide, iron oxide, copper oxide, titanium oxide, zirconium oxide, zeolite, cerium oxide, and hydrotalcite. Among these, the carrier of the catalyst (A) is preferably at least one kind selected from the group consisting of zeolite, hydrotalcite, aluminum oxide, activated carbon, titanium oxide, zirconium oxide, and cerium oxide, among which at least one kind selected from the group consisting of zeolite, aluminum oxide, and hydrotalcite is more preferred.

The shape of the carrier is not particularly limited, and is generally in the form of powder, the median diameter (d50) of which is generally 1 to 300 µm, and the shape thereof may be other shapes derived from powder depending on necessity.

The total content of the first component and the second component contained in the catalyst (A) including the carrier is preferably 10% by mass or more, more preferably 20% by mass or more, further preferably 30% by mass or more, still further preferably 35% by mass or more, still more further preferably 40% by mass or more, even further preferably 50% by mass or more, even still further preferably 55% by mass or more, and even still more further preferably 60% by mass or more, and is preferably 90% by mass or less, more preferably 85% by mass or less, further preferably 80% by mass or less, and still further preferably 75% by mass or less, from the standpoint of the yield of the Guerbet alcohol and the low selectivity of the hydrocarbon compound.

The catalyst (A) may contain the first component and the second component with no carrier included. The total content of the first component and the second component contained in the catalyst (A) including no carrier is preferably 50% by mass or more, more preferably 60% by mass or more, further preferably 65% by mass or more, and still further preferably 70% by mass or more, from the standpoint of the yield of the Guerbet alcohol and the low selectivity of the hydrocarbon compound.

The total content of the first component and the second component contained in the catalyst (A) in the case where the first component is 10% by mass or more is preferably 45% by mass or more, more preferably 50% by mass or more, and further preferably 60% by mass or more, and is preferably 90% by mass or less, more preferably 85% by mass or less, and further preferably 80% by mass or less, from the standpoint of the yield of the Guerbet alcohol and the low selectivity of the hydrocarbon compound.

The total content of the first component and the second component contained in the catalyst (A) in the case where the first component is less than 10% by mass is preferably 30% by mass or more, more preferably 40% by mass or more, and further preferably 50% by mass or more, and is preferably 80% by mass or less, and more preferably 75% by mass or less, from the same standpoint.

The total existing amount of the first component and the second component contained in the catalyst (A) in the case where the existing amount of the first component contained in the catalyst (A) is 0.3 part by mol or more per 10,000 parts by mol of the raw material alcohol is preferably 1.2 parts by mol or more, and more preferably 1.5 parts by mol or more, and is preferably 2.4 parts by mol or less, more preferably 2.2 parts by mol or less, and further preferably 2 parts by mol or less, per 10,000 parts by mol of the raw material alcohol, from the standpoint of the yield of the Guerbet alcohol and the low selectivity of the hydrocarbon compound.

The total existing amount of the first component and the second component contained in the catalyst (A) in the case where the existing amount of the first component contained in the catalyst (A) is less than 0.3 part by mol per 10,000 parts by mol of the raw material alcohol is preferably 0.8 part by mol or more, and more preferably 1.0 part by mol or more, and is preferably 1.8 parts by mol or less, and more preferably 1.5 parts by mol or less, per 10,000 parts by mol of the raw material alcohol, from the same standpoint.

The molar ratio of the first component with respect to the second component (first component/second component) in the catalyst (A) is 2.9 or less, preferably 2.7 or less, more preferably 2.5 or less, and further preferably 2.3 or less, and is preferably 0.01 or more, more preferably 0.05 or more, further preferably 0.11 or more, still further preferably 0.4 or more, still more further preferably 0.6 or more, and even further preferably 0.8 or more, from the standpoint of the yield of the Guerbet alcohol and the low selectivity of the hydrocarbon compound.

The molar ratio of the third component with respect to the total amount of the first component and the second component (third component/total of first component and second component) in the catalyst (A) is preferably less than 0.004, more preferably less than 0.003, further preferably less than 0.002, still further preferably less than 0.001, and still more further preferably less than 0.0001.

The shape of the catalyst (A) is not particularly limited, and examples thereof include powder, granules, noodle-like form, and pellets. The shapes including granules, noodle-like form, and pellets can be produced through granulation or molding the catalyst (A) in the form of powder by a known method.

In the case where the catalyst (A) is in the form of powder, the median diameter (d50) of the catalyst (A) is preferably 1 µm or more, more preferably 3 µm or more, further preferably 5 µm or more, and still further preferably 7 µm or more, from the standpoint of the recoverability, and is preferably 300 µm or less, more preferably 200 µm or less, further preferably 100 µm or less, and still further preferably 30 µm or less, from the standpoint of the yield of the Guerbet alcohol and the low selectivity of the hydrocarbon compound. The median diameter (d50) of the catalyst (A) can be measured with a laser diffraction/scattering particle size distribution analyzer "LA-920" (available from Horiba, Ltd.). The measurement may be performed after dispersing 0.05 g thereof in ion exchanged water as the measurement solvent under stirring (stirring rate: level 4), and the median diameter (d50) is calculated with an appropriate relative refractive index.

In the case where the catalyst (A) is in the form of granules, the average particle diameter of the catalyst (A) is preferably 0.2 mm or more, more preferably 0.4 mm or more, and further preferably 0.6 mm or more, from the standpoint of the recoverability, and is preferably 2.0 mm or less, more preferably 1.3 mm or less, and further preferably 0.8 mm or less, from the standpoint of the yield of the Guerbet alcohol and the low selectivity of the hydrocarbon compound. The average particle diameter of the catalyst (A) herein means the arithmetic average particle diameter, and can be obtained with a vernier caliper. The number of the granules for obtaining the average particle diameter may be 30 granules randomly selected.

In the case where the catalyst (A) is in the noodle-like form, the average diameter of the catalyst (A) is preferably 1.0 mm or more, more preferably 1.2 mm or more, and further preferably 1.4 mm or more, from the standpoint of the strength of the catalyst, and is preferably 2.5 mm or less, more preferably 2.0 mm or less, and further preferably 1.5 mm or less, from the standpoint of the yield of the Guerbet alcohol and the low selectivity of the hydrocarbon compound. The average diameter of the catalyst (A) herein means the arithmetic average diameter, and can be obtained with a vernier caliper. The number of the noodles for obtaining the average diameter may be 30 noodles randomly selected.

In the case where the catalyst (A) is in the noodle-like form, the average length of the catalyst (A) is preferably 2 mm or more, and more preferably 3 mm or more, from the standpoint of the strength of the catalyst, and is preferably 8 mm or less, more preferably 6 mm or less, and further preferably 4 mm or less, from the standpoint of the homogeneity in packing, the yield of the Guerbet alcohol, and the low selectivity of the hydrocarbon compound. The average length of the catalyst (A) herein means the arithmetic average length, and can be obtained with a vernier caliper. The number of the noodles for obtaining the average length may be 30 noodles randomly selected.

In the case where the catalyst (A) is in the form of pellets, the average diameter and the average height of the catalyst (A) each are preferably 1.5 mm or more, more preferably 2.0 mm or more, and further preferably 2.5 mm or more, from the standpoint of the strength of the catalyst, and is preferably 5.0 mm or less, more preferably 4.0 mm or less, and further preferably 3.0 mm or less, from the standpoint of the yield of the Guerbet alcohol and the low selectivity of the hydrocarbon compound. The average diameter and the average height of the catalyst (A) herein mean the arithmetic average diameter and the arithmetic average height respectively, and can be obtained with a vernier caliper. The number of the pellets for obtaining the average diameter or the average height may be 30 pellets randomly selected.

### (Preparation of Catalyst (A))

The catalyst (A) used in the present invention may be prepared by a known method, such as a precipitation method, an impregnation method, an ion exchange method, an alloying method, and an adsorption method.

The catalyst (A) may be prepared preferably by a method of supporting the first component and the second component by the precipitation method on the carrier.

The precipitation method for supporting the first component and the second component on the carrier may be performed, for example, in the following manner.

A first component-containing water-soluble salt and a second component-containing water-soluble salt are dissolved in ion exchanged water to prepare an aqueous solution containing the first component and the second component. Separately, an alkali aqueous solution containing an alkali component, such as sodium carbonate, and a slurry containing a component to be the carrier, such as zeolite, are prepared, respectively. Subsequently, the aqueous solution containing the first component and the second component is added in a dropwise manner to the slurry, simultaneously the alkali aqueous solution is added in a dropwise manner thereto, and the solutions are added in a dropwise manner thereto for a prescribed period of time while retaining a prescribed pH of the slurry for insolubilizing and depositing the first component and the second component as a carbonate salt or a hydroxide, so as to provide a solid matter including the carrier having the carbonate salt or the hydroxide of the first component and the second component attached thereto. The solid matter is repeatedly subjected to an operation including filtration and washing, and baked at a prescribed temperature for a prescribed period of time, so as to provide a baked material including the carrier having the first component and the second component supported thereon.

The baking temperature for providing the baked material including the carrier having the first and second components supported thereon is preferably 300°C or more, more preferably 350°C or more, and further preferably 400°C or more, and is preferably 900°C or less, more preferably 850°C or less, and further preferably 800°C or less, from the standpoint of the yield of the Guerbet alcohol and the low selectivity of the hydrocarbon compound.

The baking time for providing the baked material including the carrier having the first and second components supported thereon is preferably 1 hour or more, more preferably 2 hours or more, and further preferably 3 hours or more, and is preferably 10 hours or less, more preferably 7 hours or less, and further preferably 5 hours or less, from the standpoint of the yield of the Guerbet alcohol and the low selectivity of the hydrocarbon compound.

The baking atmosphere for providing the baked material including the carrier having the first and second components supported thereon is not particularly limited, examples of which include an inert gas atmosphere, such as nitrogen, an oxidizing atmosphere, such as air, and a reducing atmosphere, such as hydrogen, and among these, an oxidizing atmosphere, such as air, is preferred from the standpoint of the yield of the Guerbet alcohol and the low selectivity of the hydrocarbon compound. The baking atmosphere may be either a close state or an open state.

### [Base Catalyst (B)]

In the method of producing a Guerbet alcohol of the present invention, a base catalyst (B) is preferably used with the catalyst (A).

The use of the base catalyst (B) with the catalyst (A) can facilitate the shortening of the reaction time, the enhancement of the yield of the Guerbet alcohol formed, and the decrease of the amount of the by-produced hydrocarbon compound.

Examples of the base catalyst (B) include an alkali metal and an alkaline earth metal, and hydrides, hydroxides, carbonates, hydrogen carbonates, and alkoxides thereof.

Specific examples of hydrides, hydroxides, carbonates, hydrogen carbonates, and alkoxide compounds of an alkali metal and an alkaline earth metal include an alkali metal hydroxide, such as LiOH, NaOH, KOH, RbOH, and CsOH; an alkali metal carbonate, such as Li₂CO₃, Na₂CO₃, K₂CO₃, Rb₂CO₃, Cs₂CO₃; an alkali metal hydrogen carbonate, such as LiHCOs, NaHCOs, KHCO₃, RbHCO₃, C₃HCO₃; an alkali metal alkoxide compound, such as sodium methoxide, sodium ethoxide, sodium t-butoxide, potassium methoxide, potassium ethoxide, and potassium t-butoxide; and an alkaline earth metal hydroxide, such as Mg(OH)₂ and Ca(OH)₂.

In the base catalyst (B), an alkali metal hydroxide, such as LiOH, NaOH, KOH, RbOH, and CsOH; and an alkali metal alkoxide compound, such as sodium methoxide, sodium ethoxide, sodium t-butoxide, potassium methoxide, potassium ethoxide, and potassium t-butoxide, all of which are strong bases, are preferred from the standpoint of the yield of the Guerbet alcohol and the low selectivity of the hydrocarbon compound, among which NaOH and KOH are more preferred, and KOH is further preferred, from the standpoint of the versatility and the economic efficiency.

One kind of the base catalyst (B) may be used alone, or two or more kinds thereof may be used in combination.

The base catalyst (B) may not be supported on a carrier.

The amount of the base catalyst (B) per 100 parts by mol in total of the amount of the raw material alcohol is preferably 0.1 part by mol or more, more preferably 0.2 part by mol or more, and further preferably 0.3 part by mol or more, from the standpoint of the yield of the Guerbet alcohol and the low selectivity of the hydrocarbon compound, and is preferably 7 parts by mol or less, more preferably 5 parts by mol or less, and further preferably 3 parts by mol or less, from the standpoint of the selectivity.

### [Guerbet Reaction]

In the method of producing a Guerbet alcohol of the present invention, a raw material alcohol having 8 or more and 36 or less carbon atoms is reacted (dehydration condensation reaction (Guerbet reaction)) in the presence of the catalyst (A) including the carrier having the particular components supported thereon, so as to form a Guerbet alcohol. The catalyst (A) may be a catalyst that does not include the carrier for supporting.

The use mode of the catalyst (A) for the Guerbet reaction is not particularly limited, and may be either suspended bed reaction or fixed bed reaction, which may be appropriately selected depending on the catalyst activity, the reaction scale, and the like. The material of the reaction equipment used for the Guerbet reaction may be a stainless steel (such as SUS201, SUS202, SUS301, SUS302, SUS303, SUS304, SUS305, SUS316, SUS317, SUS329J1, SUS403, SUS405, SUS420, SUS430, SUS430LX, and SUS630), and may be glass.

The reaction mode of the method of producing a Guerbet alcohol of the present invention may be either a batch system, a semi-batch system, or a continuous system.

In the case where the reaction mode is suspended bed reaction, a batch system or a semi-batch system is preferred from the standpoint of the operability, and the amount of the catalyst (A) per 100 parts by mass in total of the amount of the raw material alcohol is preferably 0.001 part by mass or more, more preferably 0.005 part by mass or more, and further preferably 0.01 part by mass or more, from the standpoint of the yield of the Guerbet alcohol and the low selectivity of the hydrocarbon compound, and is preferably 10 parts by mass or less, more preferably 5 parts by mass or less, and further preferably 1 part by mass or less, from the standpoint of the economic efficiency.

In the case where the reaction mode is fixed bed reaction, a continuous system is preferred from the standpoint of the yield of the Guerbet alcohol and the low selectivity of the hydrocarbon compound, and the amount of the catalyst (A) per 100 parts by mass in total of the amount of the raw material alcohol is preferably 10 parts by mass or more, more preferably 15 parts by mass or more, further preferably 25 parts by mass or more, and still further preferably 50 parts by mass or more, from the standpoint of the yield of the Guerbet alcohol and the low selectivity of the hydrocarbon compound, and is preferably 4,000 parts by mass or less, more preferably 2,500 parts by mass or less, further preferably 1,000 parts by mass or less, and still further preferably 500 parts by mass or less, from the standpoint of the economic efficiency.

The reaction temperature of the Guerbet reaction may be appropriately determined in consideration of the boiling point of the raw material alcohol, is preferably 180°C or more, more preferably 190°C or more, further preferably 200°C or more, and still further preferably 220°C or more, from the standpoint of the yield of the Guerbet alcohol and the low selectivity of the hydrocarbon compound, and is preferably 300°C or less, more preferably 280°C or less, and further preferably 260°C or less, from the standpoint of the selectivity.

The reaction time of the Guerbet reaction may be appropriately determined depending on the reaction temperature and the kind of the raw material alcohol, and the reaction time in the batch system is generally 1 hour or more from the standpoint of the yield of the Guerbet alcohol and the low selectivity of the hydrocarbon compound, and is preferably 20 hours or less, and more preferably 10 hours or less, from the standpoint of the productivity. The LHSV (liquid hourly space velocity) in the continuous system is preferably 10/hr or less, more preferably 7/hr or less, further preferably 5/hr or less, and still further preferably 3/hr or less, from the standpoint of the yield of the Guerbet alcohol and the low selectivity of the hydrocarbon compound, and is preferably 0.03/hr or more, more preferably 0.05/hr or more, further preferably 0.1/hr or more, and still further preferably 0.2/hr or more, from the standpoint of the productivity.

The pressure of the gas phase in reaction of the Guerbet reaction may be either reduced pressure, ordinary pressure, or increased pressure, may be reduced pressure from the standpoint of the yield of the Guerbet alcohol and the low selectivity of the hydrocarbon compound, and may be ordinary pressure from the standpoint of the operability and the economic efficiency.

In the Guerbet reaction, it is preferred from the standpoint of the yield of the Guerbet alcohol and the low selectivity of the hydrocarbon compound that an inert gas is introduced to the reaction system, and the inert gas is allowed to flow as a carrier gas. The inert gas is not particularly limited, examples of which include nitrogen gas and argon gas, and among these, nitrogen gas is preferably used.

The inert gas may be allowed to flow by a method of flowing in the upper part of the reaction liquid, a method of bubbling in the reaction liquid, and the like.

The flow rate of the inert gas in increasing the temperature until reaching the reaction temperature is not particularly limited, and the flow rate per 1 kg of the reaction liquid is preferably 0.5 L/hr or more, more preferably 3 L/hr or more, and further preferably 8 L/hr or more, from the standpoint of the yield of the Guerbet alcohol and the low selectivity of the hydrocarbon compound, and is preferably 30 L/hr or less, more preferably 25 L/hr or less, and further preferably 20 L/hr or less, from the standpoint of the economic efficiency.

The flow rate of the inert gas in the reaction after reaching the reaction temperature is not particularly limited, and the flow rate per 1 kg of the reaction liquid is preferably 0.02 L/hr or more, more preferably 0.08 L/hr or more, and further preferably 0.1 L/hr or more, from the standpoint of the yield of the Guerbet alcohol and the low selectivity of the hydrocarbon compound, and is preferably 10 L/hr or less, more preferably 5 L/hr or less, and further preferably 2 L/hr or less, from the standpoint of the economic efficiency.

The Guerbet alcohol formed in the production method of the present invention is determined depending on the kind of the raw material alcohol used, may be saturated or unsaturated, may be primary or secondary, and may have a cyclic structure.

The number of carbon atoms of the Guerbet alcohol formed in the production method of the present invention is preferably 16 or more, more preferably 18 or more, and further preferably 20 or more, and is preferably 72 or less, more preferably 44 or less, further preferably 40 or less, and still further preferably 36 or less, from the standpoint of the yield of the Guerbet alcohol and the low selectivity of the hydrocarbon compound.

The production method of the present invention can shorten the reaction time, can enhance the yield of the Guerbet alcohol formed, and can decrease the by-produced amount of the hydrocarbon. The Guerbet alcohol obtained by the production method of the present invention can be applied directly to various purposes, and may also be applied after purification by a distillation operation or the like depending on necessity. A Guerbet alcohol is useful as a raw material or an intermediate material of surfactants, textile oil agents, fabric softeners, cosmetics, medical drugs, lubricating oils, and the like. From the standpoint of the application to these purposes, the purity of the Guerbet alcohol is preferably 95% by mass or more, more preferably 97% by mass or more, and further preferably 98% by mass or more.

In addition to the aforementioned embodiments, the present invention relates to the following methods for producing a Guerbet alcohol.
<1> A method of producing a Guerbet alcohol, including reacting a raw material alcohol having 8 or more and 36 or less carbon atoms, in the presence of a catalyst (A) containing a first component and a second component below, having a molar ratio of the first component with respect to the second component (first component/second component) of 2.9 or less:
   first component: copper, and
   second component: one kind selected from the group consisting of elements that are elements belonging to Groups 3 to 12 in the fourth to sixth periods of the periodic table, except copper and nickel.
<2> The method of producing a Guerbet alcohol according to the item <1>, wherein the molar ratio of the first component with respect to the second component (first component/second component) in the catalyst (A) is preferably 0.01 or more and 2.9 or less.
<3> The method of producing a Guerbet alcohol according to the item <1> or <2>, wherein the molar ratio of the first component with respect to the second component (first component/second component) in the catalyst (A) is preferably 0.05 or more and 2.7 or less.
<4> The method of producing a Guerbet alcohol according to any one of the items <1> to <3>, wherein the molar ratio of the first component with respect to the second component (first component/second component) in the catalyst (A) is preferably 0.11 or more and 2.5 or less.
<5> The method of producing a Guerbet alcohol according to any one of the items <1> to <4>, wherein the molar ratio of the first component with respect to the second component (first component/second component) in the catalyst (A) is preferably 0.4 or more, more preferably 0.6 or more, and further preferably 0.8 or more, and is preferably 2.3 or less.
<6> The method of producing a Guerbet alcohol according to any one of the items <1> to <5>, wherein the content of the first component contained in the catalyst (A) is preferably 4% by mass or more and 55% by mass or less.
<7> The method of producing a Guerbet alcohol according to any one of the items <1> to <6>, wherein the content of the first component contained in the catalyst (A) is preferably 6% by mass or more and 50% by mass or less.
<8> The method of producing a Guerbet alcohol according to any one of the items <1> to <7>, wherein the content of the first component contained in the catalyst (A) is preferably 7% by mass or more and 45% by mass or less.
<9> The method of producing a Guerbet alcohol according to any one of the items <1> to <8>, wherein the content of the first component contained in the catalyst (A) is preferably 10% by mass or more and 40% by mass or less.
<10> The method of producing a Guerbet alcohol according to any one of the items <1> to <9>, wherein the average primary particle diameter of the first component contained in the catalyst (A) is preferably 0.2 nm or more and 50 nm or less.
<11> The method of producing a Guerbet alcohol according to any one of the items <1> to <10>, wherein the average primary particle diameter of the first component contained in the catalyst (A) is preferably 1 nm or more and 40 nm or less.
<12> The method of producing a Guerbet alcohol according to any one of the items <1> to <11>, wherein the average primary particle diameter of the first component contained in the catalyst (A) is preferably 3 nm or more and 30 nm or less.
<13> The method of producing a Guerbet alcohol according to any one of the items <1> to <12>, wherein the content of the second component contained in the catalyst (A) is preferably 3% by mass or more and 75% by mass or less.
<14> The method of producing a Guerbet alcohol according to any one of the items <1> to <13>, wherein the content of the second component contained in the catalyst (A) is preferably 5% by mass or more and 70% by mass or less.
<15> The method of producing a Guerbet alcohol according to any one of the items <1> to <14>, wherein the content of the second component contained in the catalyst (A) is preferably 10% by mass or more and 65% by mass or less.
<16> The method of producing a Guerbet alcohol according to any one of the items <1> to <15>, wherein the content of the second component contained in the catalyst (A) is preferably 15% by mass or more and 50% by mass or less.
<17> The method of producing a Guerbet alcohol according to any one of the items <1> to <15>, wherein the content of the second component contained in the catalyst (A) is preferably 15% by mass or more and 45% by mass or less.
<18> The method of producing a Guerbet alcohol according to any one of the items <1> to <15>, wherein the content of the second component contained in the catalyst (A) is preferably 15% by mass or more and 40% by mass or less.
<19> The method of producing a Guerbet alcohol according to any one of the items <1> to <18>, wherein the catalyst (A) is preferably a catalyst including a carrier having the first component and the second component supported thereon.
<20> The method of producing a Guerbet alcohol according to the item <19>, wherein the carrier of the catalyst (A) is preferably at least one kind selected from the group consisting of zeolite, aluminum oxide, and hydrotalcite.
<21> The method of producing a Guerbet alcohol according to the item <19> or <20>, wherein the total content of the first component and the second component contained in the catalyst (A) including the carrier is preferably 10% by mass or more and 90% by mass or less.
<22> The method of producing a Guerbet alcohol according to the items <19> to <21>, wherein the total content of the first component and the second component contained in the catalyst (A) including the carrier is preferably 20% by mass or more and 85% by mass or less.
<23> The method of producing a Guerbet alcohol according to any one of the items <19> to <22>, wherein the total content of the first component and the second component contained in the catalyst (A) including the carrier is preferably 30% by mass or more and 80% by mass or less.
<24> The method of producing a Guerbet alcohol according to any one of the items <19> to <23>, wherein the total content of the first component and the second component contained in the catalyst (A) including the carrier is preferably 35% by mass or more and 75% by mass or less.
<25> The method of producing a Guerbet alcohol according to the items <19> to <23>, wherein the total content of the first component and the second component contained in the catalyst (A) including the carrier is preferably 40% by mass or more and 75% by mass or less.
<26> The method of producing a Guerbet alcohol according to the items <19> to <23>, wherein the total content of the first component and the second component contained in the catalyst (A) including the carrier is preferably 50% by mass or more and 75% by mass or less.
<27> The method of producing a Guerbet alcohol according to the items <19> to <23>, wherein the total content of the first component and the second component contained in the catalyst (A) including the carrier is preferably 55% by mass or more and 75% by mass or less.
<28> The method of producing a Guerbet alcohol according to the items <19> to <23>, wherein the total content of the first component and the second component contained in the catalyst (A) including the carrier is preferably 60% by mass or more and 75% by mass or less.
<29> The method of producing a Guerbet alcohol according to any one of the items <1> to <28>, wherein the content of the third component contained in the catalyst (A) is preferably 10% by mass or less, more preferably 5% by mass or less, further preferably 3% by mass or less, still further preferably 1% by mass or less, still more further preferably 0.6% by mass or less, even further preferably 0.3% by mass or less, even still further preferably 0.1% by mass or less, even still more further preferably 0.01% by mass or less, and yet further preferably 0% by mass.
<30> The method of producing a Guerbet alcohol according to the items <1> to <29>, wherein the molar ratio of the third component with respect to the total amount of the first component and the second component (third component/total of first component and second component) in the catalyst (A) is preferably less than 0.004, more preferably less than 0.003, further preferably less than 0.002, still further preferably less than 0.001, and still more further preferably less than 0.0001.
<31> The method of producing a Guerbet alcohol according to the items <1> to <30>, wherein the first component contained in the catalyst (A) is 10% by mass or more, and the total content of the first component and the second component contained in the catalyst (A) is preferably 45% by mass or more, more preferably 50% by mass or more, and further preferably 60% by mass or more, and is preferably 90% by mass or less, more preferably 85% by mass or less, and further preferably 80% by mass or less.
<32> The method of producing a Guerbet alcohol according to the items <1> to <31>, wherein the first component contained in the catalyst (A) is less than 10% by mass, and the total content of the first component and the second component contained in the catalyst (A) is preferably 30% by mass or more, more preferably 40% by mass or more, and further preferably 50% by mass or more, and is preferably 80% by mass or less, and more preferably 75% by mass or less.
<33> The method of producing a Guerbet alcohol according to the items <1> to <32>, wherein the existing amount of the first component contained in the catalyst (A) is 0.3 part by mol or more per 10,000 parts by mol of the raw material alcohol, and the total existing amount of the first component and the second component contained in the catalyst (A) is preferably 1.2 parts by mol or more, and more preferably 1.5 parts by mol or more, and is preferably 2.4 parts by mol or less, more preferably 2.2 parts by mol or less, and further preferably 2 parts by mol or less, per 10,000 parts by mol of the raw material alcohol.
<34> The method of producing a Guerbet alcohol according to the items <1> to <33>, wherein the existing amount of the first component contained in the catalyst (A) is less than 0.3 part by mol per 10,000 parts by mol of the raw material alcohol, and the total existing amount of the first component and the second component contained in the catalyst (A) is preferably 0.8 part by mol or more, and more preferably 1.0 part by mol or more, and is preferably 1.8 parts by mol or less, and more preferably 1.5 parts by mol or less, per 10,000 parts by mol of the raw material alcohol.
<35> The method of producing a Guerbet alcohol according to any one of the items <1> to <34>, wherein a base catalyst (B) is preferably used with the catalyst (A).
<36> The method of producing a Guerbet alcohol according to the item <35>, wherein the base catalyst (B) is preferably NaOH or KOH.
<37> The method of producing a Guerbet alcohol according to the item <36>, wherein the amount of the base catalyst (B) per 100 parts by mol in total of the amount of the raw material alcohol is preferably 0.2 part by mol or more and 5 parts by mol or less.
<38> The method of producing a Guerbet alcohol according to the item <36> or <37>, wherein the amount of the base catalyst (B) per 100 parts by mol in total of the amount of the raw material alcohol is preferably 0.3 part by mol or more and 3 parts by mol or less.
<39> The method of producing a Guerbet alcohol according to any one of the items <1> to <38>, wherein the raw material alcohol is preferably a saturated linear primary aliphatic alcohol having 8 or more and 18 or less carbon atoms.
<40> The method of producing a Guerbet alcohol according to any one of the items <1> to <39>, wherein the raw material alcohol is preferably a saturated linear primary aliphatic alcohol having 10 or more and 16 or less carbon atoms.
<41> The method of producing a Guerbet alcohol according to any one of the items <1> to <39>, wherein the raw material alcohol is preferably a saturated linear primary aliphatic alcohol having 10 or more and 14 or less carbon atoms.
<42> The method of producing a Guerbet alcohol according to any one of the items <1> to <39>, wherein the raw material alcohol is preferably a saturated linear primary aliphatic alcohol having 10 or more and 12 or less carbon atoms.
<43> The method of producing a Guerbet alcohol according to any one of the items <1> to <42>, wherein the number of carbon atoms of the Guerbet alcohol is preferably 16 or more and 44 or less.
<44> The method of producing a Guerbet alcohol according to any one of the items <1> to <43>, wherein the number of carbon atoms of the Guerbet alcohol is preferably 18 or more and 40 or less.
<45> The method of producing a Guerbet alcohol according to any one of the items <1> to <44>, wherein the number of carbon atoms of the Guerbet alcohol is preferably 20 or more and 36 or less.

### Examples

The present invention will be described in more detail with reference to examples, but the present invention is not limited thereto. The measurements and evaluations in Preparation Examples, Comparative Preparation Examples, Examples, and Comparative Examples were performed in the following manner.

### (1) Measurement by ICP Emission Spectroscopy

The first component (Cu), the second component, and the third component contained in the catalysts were quantitatively determined by the ICP emission spectroscopy (high frequency inductively coupled plasma emission spectroscopy: ICP-AES, ICP-OES) with an ICP emission spectral analyzer (product name: iCAP 6500 Duo, available from Thermo Fisher Scientific, Inc.).

### (2) Measurement of Conversion of Raw Material Alcohol, Yield of Guerbet Alcohol Compound formed, and Selectivity of by-produced Hydrocarbon Compound

In Examples and Comparative Examples, the solution after completing the reaction was diluted with hexane, and then the products were quantitatively determined by analyzing by gas chromatography (column: Ultra ALLOY-1 (MS/HT) capillary column 30.0 m × 250 µm (Frontier Laboratories, Ltd.), detector: FID, injection temperature: 350°C, detector temperature: 350°C, He flow rate: 4.6 mL/min).

The conversion of the raw material alcohol, the yield of the Guerbet alcohol compound formed, and the selectivity of the by-produced hydrocarbon compound were calculated from the result of gas chromatography according to the following expressions, respectively. The results are shown in Tables 1 to 4.

The Guerbet alcohol compound means not only the Guerbet alcohol, but also the aldehyde dimer and the allyl alcohol dimer becoming the Guerbet alcohol in the subsequent hydrogenation step. Conversion of raw material alcohol (%) = 100 - (residual amount of raw material alcohol (mol)/charged amount of raw material alcohol (mol)) × 100 Yield of Guerbet alcohol compound formed (%) = ((amount of Guerbet alcohol formed (mol) + amount of aldehyde dimer formed (mol) + amount of allyl alcohol dimer formed (mol)) × 2/charged amount of raw material alcohol (mol)) × 100 Yield of by-produced hydrocarbon compound (%) = (amount of by-produced hydrocarbon compound (mol)/charged amount of raw material alcohol (mol)) × 100 Selectivity of by-produced hydrocarbon compound (%) = yield of by-produced hydrocarbon compound (%)/conversion of raw material alcohol (%)) × 100

The Guerbet alcohol formed in the case where 1-decanol (C10) is used as the raw material alcohol is C20 Guerbet alcohol, and the by-produced hydrocarbon compound is C19 alkane and C20 alkane. C19 alkane and C20 alkane show an alkane having 19 carbon atoms and an alkane having 20 carbon atoms, respectively.

The Guerbet alcohol formed in the case where 1-dodecanol (C12) is used as the raw material alcohol is C24 Guerbet alcohol, and the by-produced hydrocarbon compound is C23 alkane and C24 alkane. C23 alkane and C24 alkane show an alkane having 23 carbon atoms and an alkane having 24 carbon atoms, respectively.

The Guerbet alcohol formed in the case where 1-hexadecanol (C16) is used as the raw material alcohol is C32 Guerbet alcohol, and the by-produced hydrocarbon compound is C31 alkane and C32 alkane. C31 alkane and C32 alkane show an alkane having 31 carbon atoms and an alkane having 32 carbon atoms, respectively.

### (Comparative Preparation Example 1)

### <Preparation of Cu-Zn/zeolite by Precipitation Method>

15 g of cupric nitrate trihydrate (available from Kanto Chemical Co., Inc.) and 6 g of zinc nitrate hexahydrate (available from Fujifilm Wako Pure Chemical Corporation) were placed in a 250 mL beaker, to which 78 g of ion exchanged water was added for dissolving to prepare a metal salt aqueous solution. Subsequently, 16 g of sodium carbonate (available from Fujifilm Wako Pure Chemical Corporation) was placed in another 250 mL beaker, to which 88 g of ion exchanged water was added for dissolving to prepare a sodium carbonate aqueous solution. Furthermore, 5 g of synthetic zeolite (available from Fujifilm Wako Pure Chemical Corporation) was placed in still another 500 mL beaker, to which 78 g of ion exchanged water was added to prepare a slurry of synthetic zeolite.

The metal salt aqueous solution was added in a dropwise manner to the slurry of synthetic zeolite, and simultaneously the sodium carbonate aqueous solution was added in a dropwise manner thereto, while retaining the pH to 7 (20°C) over 27 minutes. After completing the dropwise addition, a precipitate (i.e., a solid matter including synthetic zeolite to be a carrier having Cu and a carbonate or a hydroxide of Zn attached thereto) was filtrated under reduced pressure, and the resulting cake was washed with 400 mL of ion exchanged water. The cake was subjected four times to an operation including re-slurrying, filtration under reduced pressure, and washing with water, then dried at 120°C for 16 hours, and further baked in air at 500°C for 3 hours, so as to provide a Cu-Zn/zeolite baked material (powder) (catalyst a1).

The contents of Cu and Zn contained in the resulting Cu-Zn/zeolite baked material obtained by the ICP emission spectroscopy were 34.2% by mass for the content of Cu and 11.8% by mass for the content of Zn.

### (Preparation Example 1)

### <Preparation of Cu-Zn/zeolite by Precipitation Method>

15 g of cupric nitrate trihydrate (available from Kanto Chemical Co., Inc.) and 9 g of zinc nitrate hexahydrate (available from Fujifilm Wako Pure Chemical Corporation) were placed in a 250 mL beaker, to which 78 g of ion exchanged water was added for dissolving to prepare a metal salt aqueous solution. Subsequently, 18 g of sodium carbonate (available from Fujifilm Wako Pure Chemical Corporation) was placed in another 250 mL beaker, to which 99 g of ion exchanged water was added for dissolving to prepare a sodium carbonate aqueous solution. Furthermore, 4 g of synthetic zeolite (available from Fujifilm Wako Pure Chemical Corporation) was placed in still another 500 mL beaker, to which 67 g of ion exchanged water was added to prepare a slurry of synthetic zeolite.

The same operation as in Comparative Preparation Example 1 was performed except that the metal salt aqueous solution was added in a dropwise manner to the slurry of synthetic zeolite, and simultaneously the sodium carbonate aqueous solution was added in a dropwise manner thereto, while retaining the pH to 7 (20°C) over 26 minutes, so as to provide a Cu-Zn/zeolite baked material (powder) (catalyst A1).

The contents of Cu and Zn contained in the resulting Cu-Zn/zeolite baked material obtained by the ICP emission spectroscopy were 34.7% by mass for the content of Cu and 17.4% by mass for the content of Zn.

### (Preparation Example 2)

### <Preparation of Cu-Zn/zeolite by Precipitation Method>

15 g of cupric nitrate trihydrate (available from Kanto Chemical Co., Inc.) and 18 g of zinc nitrate hexahydrate (available from Fujifilm Wako Pure Chemical Corporation) were placed in a 250 mL beaker, to which 78 g of ion exchanged water was added for dissolving to prepare a metal salt aqueous solution. Subsequently, 24 g of sodium carbonate (available from Fujifilm Wako Pure Chemical Corporation) was placed in another 250 mL beaker, to which 132 g of ion exchanged water was added for dissolving to prepare a sodium carbonate aqueous solution. Furthermore, 2 g of synthetic zeolite (available from Fujifilm Wako Pure Chemical Corporation) was placed in still another 500 mL beaker, to which 82 g of ion exchanged water was added to prepare a slurry of synthetic zeolite.

The same operation as in Comparative Preparation Example 1 was performed except that the metal salt aqueous solution was added in a dropwise manner to the slurry of synthetic zeolite, and simultaneously the sodium carbonate aqueous solution was added in a dropwise manner thereto, while retaining the pH to 7 (20°C) over 27 minutes, so as to provide a Cu-Zn/zeolite baked material (powder) (catalyst A2).

The contents of Cu and Zn contained in the resulting Cu-Zn/zeolite baked material obtained by the ICP emission spectroscopy were 31.0% by mass for the content of Cu and 32.6% by mass for the content of Zn.

### (Preparation Example 3)

### <Preparation of Cu-Zn/zeolite by Precipitation Method>

4 g of cupric nitrate trihydrate (available from Kanto Chemical Co., Inc.) and 14 g of zinc nitrate hexahydrate (available from Fujifilm Wako Pure Chemical Corporation) were placed in a 250 mL beaker, to which 80 g of ion exchanged water was added for dissolving to prepare a metal salt aqueous solution. Subsequently, 12 g of sodium carbonate (available from Fujifilm Wako Pure Chemical Corporation) was placed in another 250 mL beaker, to which 68 g of ion exchanged water was added for dissolving to prepare a sodium carbonate aqueous solution. Furthermore, 6 g of synthetic zeolite (available from Fujifilm Wako Pure Chemical Corporation) was placed in still another 500 mL beaker, to which 103 g of ion exchanged water was added to prepare a slurry of synthetic zeolite.

The same operation as in Comparative Preparation Example 1 was performed except that the metal salt aqueous solution was added in a dropwise manner to the slurry of synthetic zeolite, and simultaneously the sodium carbonate aqueous solution was added in a dropwise manner thereto, while retaining the pH to 7 (20°C) over 25 minutes, so as to provide a Cu-Zn/zeolite baked material (powder) (catalyst A3).

The contents of Cu and Zn contained in the resulting Cu-Zn/zeolite baked material obtained by the ICP emission spectroscopy were 8.8% by mass for the content of Cu and 27.7% by mass for the content of Zn.

### (Preparation Example 4)

### <Preparation of Cu-Zn/zeolite by Precipitation Method>

4 g of cupric nitrate trihydrate (available from Kanto Chemical Co., Inc.) and 24 g of zinc nitrate hexahydrate (available from Fujifilm Wako Pure Chemical Corporation) were placed in a 250 mL beaker, to which 122 g of ion exchanged water was added for dissolving to prepare a metal salt aqueous solution. Subsequently, 19 g of sodium carbonate (available from Fujifilm Wako Pure Chemical Corporation) was placed in another 250 mL beaker, to which 104 g of ion exchanged water was added for dissolving to prepare a sodium carbonate aqueous solution. Furthermore, 4 g of synthetic zeolite (available from Fujifilm Wako Pure Chemical Corporation) was placed in still another 500 mL beaker, to which 69 g of ion exchanged water was added to prepare a slurry of synthetic zeolite.

The same operation as in Comparative Preparation Example 1 was performed except that the metal salt aqueous solution was added in a dropwise manner to the slurry of synthetic zeolite, and simultaneously the sodium carbonate aqueous solution was added in a dropwise manner thereto, while retaining the pH to 7 (20°C) over 39 minutes, so as to provide a Cu-Zn/zeolite baked material (powder) (catalyst A4).

The contents of Cu and Zn contained in the resulting Cu-Zn/zeolite baked material obtained by the ICP emission spectroscopy were 8.0% by mass for the content of Cu and 42.5% by mass for the content of Zn.

### (Preparation Example 5)

### <Preparation of Cu-Zn/zeolite by Precipitation Method>

4 g of cupric nitrate trihydrate (available from Kanto Chemical Co., Inc.) and 39 g of zinc nitrate hexahydrate (available from Fujifilm Wako Pure Chemical Corporation) were placed in a 250 mL beaker, to which 184 g of ion exchanged water was added for dissolving to prepare a metal salt aqueous solution. Subsequently, 28 g of sodium carbonate (available from Fujifilm Wako Pure Chemical Corporation) was placed in another 250 mL beaker, to which 156 g of ion exchanged water was added for dissolving to prepare a sodium carbonate aqueous solution. Furthermore, 0.3 g of synthetic zeolite (available from Fujifilm Wako Pure Chemical Corporation) was placed in still another 500 mL beaker, to which 80 g of ion exchanged water was added to prepare a slurry of synthetic zeolite.

The same operation as in Comparative Preparation Example 1 was performed except that the metal salt aqueous solution was added in a dropwise manner to the slurry of synthetic zeolite, and simultaneously the sodium carbonate aqueous solution was added in a dropwise manner thereto, while retaining the pH to 7 (20°C) over 26 minutes, so as to provide a Cu-Zn/zeolite baked material (powder) (catalyst A5).

The contents of Cu and Zn contained in the resulting Cu-Zn/zeolite baked material obtained by the ICP emission spectroscopy were 7.2% by mass for the content of Cu and 63.1% by mass for the content of Zn.

### (Preparation Example 6)

### <Preparation of Cu-Zn by Precipitation Method>

18 g of cupric nitrate trihydrate (available from Kanto Chemical Co., Inc.) and 22 g of zinc nitrate hexahydrate (available from Fujifilm Wako Pure Chemical Corporation) were placed in a 250 mL beaker, to which 93 g of ion exchanged water was added for dissolving to prepare a metal salt aqueous solution. Subsequently, 28 g of sodium carbonate (available from Fujifilm Wako Pure Chemical Corporation) was placed in another 250 mL beaker, to which 158 g of ion exchanged water was added for dissolving to prepare a sodium carbonate aqueous solution. Furthermore, 60 g of ion exchanged water was placed in still another 500 mL beaker to prepare ion exchanged water without adding synthetic zeolite.

The same operation as in Comparative Preparation Example 1 was performed except that the metal salt aqueous solution was added in a dropwise manner to the ion exchanged water having no synthetic zeolite added thereto, and simultaneously the sodium carbonate aqueous solution was added in a dropwise manner thereto, while retaining the pH to 7 (20°C) over 39 minutes, so as to provide a Cu-Zn baked material (powder) (catalyst A6).

The contents of Cu and Zn contained in the resulting Cu-Zn baked material obtained by the ICP emission spectroscopy were 37.0% by mass for the content of Cu and 38.3% by mass for the content of Zn.

### (Preparation Example 7)

### <Preparation of Cu-Zn/HT by Precipitation Method>

The same operation as in Preparation Example 2 was performed except that hydrotalcite (product name: Kyowaad 500PL, available from Kyowa Chemical Industry Co., Ltd.) was used instead of the synthetic zeolite (available from Fujifilm Wako Pure Chemical Corporation), so as to provide a Cu-Zn/HT baked material (powder) (catalyst A7).

The contents of Cu and Zn contained in the resulting Cu-Zn/HT baked material obtained by the ICP emission spectroscopy were 33.7% by mass for the content of Cu and 38.5% by mass for the content of Zn.

### (Preparation Example 8)

### <Preparation of Cu-Zn/Al₂O₃ by Precipitation Method>

The same operation as in Preparation Example 2 was performed except that aluminum oxide (product name: GP-20, available from Mizusawa Industrial Chemicals, Ltd.) was used instead of the synthetic zeolite (available from Fujifilm Wako Pure Chemical Corporation), so as to provide a Cu-Zn/Al₂O₃ baked material (powder) (catalyst A8).

The contents of Cu and Zn contained in the resulting Cu-Zn/Al₂O₃ baked material obtained by the ICP emission spectroscopy were 36.2% by mass for the content of Cu and 36.5% by mass for the content of Zn.

### [Examples 1 and 2 and Comparative Example 1]

### Investigation into Molar Ratio (First Component/Second Component)

### (Example 1)

In a 1 L five-neck glass flask equipped with a stirrer, a thermometer, a nitrogen blowing tube, a sampling tube, and a condenser and a dephlegmator for isolating by-produced water in reaction, 600.0 g (3.79 mol) of 1-decanol (C10) (product name: Kalcol 1098, available from Kao Corporation) as the raw material alcohol, 13.3 g (3 parts by mol per 100 parts by mol in total of the amount of the raw material alcohol) of a 48% potassium hydroxide aqueous solution (available from Kanto Chemical Co., Inc.) as the base catalyst (B), and 0.06 g (0.01 part by mass per 100 parts by mass in total of the amount of the raw material alcohol) of the Cu-Zn/zeolite baked material prepared in Preparation Example 1 as the catalyst (A) were charged, and the system was heated while bubbling nitrogen gas into the system at a flow rate of 6 L/hr. After the time when the temperature in the system reached 225°C, the flow rate of nitrogen gas was changed to 0.13 L/hr, and the reaction was performed for 8 hours. The results are shown in Table 1.

### (Example 2 and Comparative Example 1)

The reaction was performed in the same manner as in Example 1 except that the catalyst was changed as shown in Table 1. The results are shown in Table 1.

### [Examples 3 to 5]

### Investigation into Molar Ratio (First Component/Second Component)

The reaction was performed in the same manner as in Example 1 except that the catalyst was changed as shown in Table 2. The results are shown in Table 2.

### [Examples 6 to 8]

### Investigation into Carrier

The reaction was performed in the same manner as in Example 1 except that the catalyst was changed as shown in Table 3. The results are shown in Table 3.

### [Examples 9 and 10]

### Investigation into Raw Material Alcohol

The reaction was performed in the same manner as in Example 1 except that the catalyst, the raw material alcohol, and the reaction temperature were changed as shown in Table 4. The results are shown in Table 4.

**Table 1: Investigation into Molar Ratio (First Component/Second Component)**

| | | | Example 1 | Example 2 | Comparative Example 1 |
|---|---|---|---|---|---|
| Raw material alcohol | | | 1-decanol (C10) | 1-decanol (C10) | 1-decanol (C10) |
| Base catalyst (B) | Kind | | KOH | KOH | KOH |
| | Addition amount (part by mol per 100 parts by mol of raw material alcohol) | | 3 | 3 | 3 |
| Catalyst | Kind | | Catalyst A1 | Catalyst A2 | Catalyst a1 |
| | Addition amount (part by mass per 100 parts by mass of raw material alcohol) | | 0.01 | 0.01 | 0.01 |
| | First component | Kind | Cu | Cu | Cu |
| | | Content (% by mass) | 34.7 | 31.0 | 34.2 |
| | | Atomic weight (g/mol) | 63.5 | 63.5 | 63.5 |
| | | Existing amount (part by mol per 10,000 parts by mol of raw material alcohol) | 0.865 | 0.773 | 0.852 |
| | Second component | Kind | Zn | Zn | Zn |
| | | Content (% by mass) | 17.4 | 32.6 | 11.8 |
| | | Atomic weight (g/mol) | 65.4 | 65.4 | 65.4 |
| | | Existing amount (part by mol per 10,000 parts by mol of raw material alcohol) | 0.421 | 0.789 | 0.286 |
| | Carrier | Kind | zeolite | zeolite | zeolite |
| | Total content of first component and second component (% by mass) | | 52.1 | 63.6 | 46.0 |
| | Total existing amount of first component and second component (part by mol per 10,000 parts by mol of raw material alcohol) | | 1.286 | 1.562 | 1.138 |
| | Molar ratio (first component/second component) * | | 2.05 | 0.98 | 2.99 |
| Reaction condition | Reaction temperature (°C) | | 225 | 225 | 225 |
| | Reaction time (h) | | 8 | 8 | 8 |
| Evaluation | Conversion of raw material alcohol (%) | | 66 | 71 | 34 |
| | Yield of Guerbet alcohol compound formed (%) | | 61 | 66 | 32 |
| | Selectivity of by-produced hydrocarbon compound (%) | | 1.6 | 1.2 | 2.4 |

| | | | | | |
|---|---|---|---|---|---|
| * Molar ratio (first component/second component) = (content of first component/atomic weight of first component)/(content of second component/atomic weight of second component) | | | | | |

**Table 2: Investigation into Molar Ratio (First Component/Second Component)**

| | | | Example 3 | Example 4 | Example 5 |
|---|---|---|---|---|---|
| Raw material alcohol | | | 1-decanol (C10) | 1-decanol (C10) | 1-decanol (C10) |
| Base catalyst (B) | Kind | | KOH | KOH | KOH |
| | Addition amount (part by mol per 100 parts by mol of raw material alcohol) | | 3 | 3 | 3 |
| Catalyst | Kind | | Catalyst A3 | Catalyst A4 | Catalyst A5 |
| | Addition amount (part by mass per 100 parts by mass of raw material alcohol) | | 0.01 | 0.01 | 0.01 |
| | First component | Kind | Cu | Cu | Cu |
| | | Content (% by mass) | 8.8 | 8.0 | 7.2 |
| | | Atomic weight (g/mol) | 63.5 | 63.5 | 63.5 |
| | | Existing amount (part by mol per 10,000 parts by mol of raw material alcohol) | 0.219 | 0.199 | 0.179 |
| | Second component | Kind | Zn | Zn | Zn |
| | | Content (% by mass) | 27.7 | 42.5 | 63.1 |
| | | Atomic weight (g/mol) | 65.4 | 65.4 | 65.4 |
| | | Existing amount (part by mol per 10,000 parts by mol of raw material alcohol) | 0.670 | 1.029 | 1.527 |
| | Carrier | Kind | zeolite | zeolite | zeolite |
| | Total content of first component and second component (% by mass) | | 36.5 | 50.5 | 70.3 |
| | Total existing amount of first component and second component (part by mol per 10,000 parts by mol of raw material alcohol) | | 0.889 | 1.228 | 1.706 |
| | Molar ratio (first component/second component) * | | 0.33 | 0.19 | 0.12 |
| Reaction condition | Reaction temperature (°C) | | 225 | 225 | 225 |
| | Reaction time (h) | | 8 | 8 | 8 |
| Evaluation | Conversion of raw material alcohol (%) | | 39 | 61 | 46 |
| | Yield of Guerbet alcohol compound formed (%) | | 36 | 57 | 43 |
| | Selectivity of by-produced hydrocarbon compound (%) | | 2.1 | 1.6 | 2.1 |

| | | | | | |
|---|---|---|---|---|---|
| * Molar ratio (first component/second component) = (content of first component/atomic weight of first component)/(content of second component/atomic weight of second component) | | | | | |

**Table 3: Investigation into Carrier**

| | | | Example 6 | Example 7 | Example 8 |
|---|---|---|---|---|---|
| Raw material alcohol | | | 1-decanol (C10) | 1-decanol (C10) | 1-decanol (C10) |
| Base catalyst (B) | Kind | | KOH | KOH | KOH |
| | Addition amount (part by mol per 100 parts by mol of raw material alcohol) | | 3 | 3 | 3 |
| Catalyst | Kind | | Catalyst A6 | Catalyst A7 | Catalyst A8 |
| | Addition amount (part by mass per 100 parts by mass of raw material alcohol) | | 0.01 | 0.01 | 0.01 |
| | First component | Kind | Cu | Cu | Cu |
| | | Content (% by mass) | 37.0 | 33.7 | 36.2 |
| | | Atomic weight (g/mol) | 63.5 | 63.5 | 63.5 |
| | | Existing amount (part by mol per 10,000 parts by mol of raw material alcohol) | 0.922 | 0.840 | 0.902 |
| | Second component | Kind | Zn | Zn | Zn |
| | | Content (% by mass) | 38.3 | 38.5 | 36.5 |
| | | Atomic weight (g/mol) | 65.4 | 65.4 | 65.4 |
| | | Existing amount (part by mol per 10,000 parts by mol of raw material alcohol) | 0.927 | 0.932 | 0.883 |
| | Carrier | Kind | - | HT | Al₂O₃ |
| | Total content of first component and second component (% by mass) | | 75.3 | 72.2 | 72.7 |
| | Total existing amount of first component and second component (part by mol per 10,000 parts by mol of raw material alcohol) | | 1.849 | 1.772 | 1.786 |
| | Molar ratio (first component/second component) * | | 0.99 | 0.90 | 1.02 |
| Reaction condition | Reaction temperature (°C) | | 225 | 225 | 225 |
| | Reaction time (h) | | 8 | 8 | 8 |
| Evaluation | Conversion of raw material alcohol (%) | | 71 | 74 | 70 |
| | Yield of Guerbet alcohol compound formed (%) | | 65 | 68 | 64 |
| | Selectivity of by-produced hydrocarbon compound (%) | | 1.2 | 1.3 | 1.2 |

| | | | | | |
|---|---|---|---|---|---|
| * Molar ratio (first component/second component) = (content of first component/atomic weight of first component)/(content of second component/atomic weight of second component) | | | | | |

**Table 4: Investigation into Raw Material Alcohol**

| | | | Example 9 | Example 10 |
|---|---|---|---|---|
| Raw material alcohol | | | 1-dodecanol (C12) | 1-hexadecanol (C16) |
| Base catalyst (B) | Kind | | KOH | KOH |
| | Addition amount (part by mol per 100 parts by mol of raw material alcohol) | | 3 | 3 |
| Catalyst | Kind | | Catalyst A2 | Catalyst A2 |
| | Addition amount (part by mass per 100 parts by mass of raw material alcohol) | | 0.01 | 0.01 |
| | First component | Kind | Cu | Cu |
| | | Content (% by mass) | 31.0 | 31.0 |
| | | Atomic weight (g/mol) | 63.5 | 63.5 |
| | | Existing amount (part by mol per 10,000 parts by mol of raw material alcohol) | 0.910 | 1.184 |
| | Second component | Kind | Zn | Zn |
| | | Content (% by mass) | 32.6 | 32.6 |
| | | Atomic weight (g/mol) | 65.4 | 65.4 |
| | | Existing amount (part by mol per 10,000 parts by mol of raw material alcohol) | 0.929 | 1.208 |
| | Carrier | Kind | zeolite | zeolite |
| | Total content of first component and second component (% by mass) | | 63.6 | 63.6 |
| | Total existing amount of first component and second component (part by mol per 10,000 parts by mol of raw material alcohol) | | 1.839 | 2.392 |
| | Molar ratio (first component/second component) * | | 0.98 | 0.98 |
| Reaction condition | Reaction temperature (°C) | | 240 | 240 |
| | Reaction time (h) | | 8 | 8 |
| Evaluation | Conversion of raw material alcohol (%) | | 73 | 28 |
| | Yield of Guerbet alcohol compound formed (%) | | 66 | 26 |
| | Selectivity of by-produced hydrocarbon compound (%) | | 2.3 | 4.0 |

| | | | | |
|---|---|---|---|---|
| * Molar ratio (first component/second component) = (content of first component/atomic weight of first component)/(content of second component/atomic weight of second component) | | | | |

### (Summary of Results 1)

### Table 1: Investigation into Molar Ratio (First Component/Second Component)

The catalyst a1 used in Comparative Example 1 has the approximately same content of the first component as the catalysts A1 and A2 used in Examples 1 and 2, but has a molar ratio (first component/second component) that deviates from the scope of the present invention. It is found that Examples 1 and 2 using the catalysts A1 and A2 each have a low selectivity of the by-produced hydrocarbon compound, and are excellent in the effect of enhancing the yield of the Guerbet alcohol compound formed, as compared to Comparative Example 1 using the catalyst a1.

### (Summary of Results 2)

### Table 2: Investigation into Molar Ratio (First Component/Second Component)

It is found that Examples 3 to 5 using the catalysts A3 to A5 each have a low selectivity of the by-produced hydrocarbon compound, and are excellent in the effect of enhancing the yield of the Guerbet alcohol compound formed, as compared to Comparative Example 1 using the catalyst a1.

### (Summary of Results 3)

### Table 3: Investigation into Carrier

While the catalyst A6 used in Example 6 has no carrier, it is found that Examples 6 using the catalyst A6 has a low selectivity of the by-produced hydrocarbon compound, and is excellent in the effect of enhancing the yield of the Guerbet alcohol compound formed, as compared to Comparative Example 1 using the catalyst a1.

It is found that Example 7 using the catalyst A7 including hydrotalcite (HT) as the carrier and Example 8 using the catalyst A8 including aluminum oxide (Al₂O₃) as the carrier each have a low selectivity of the by-produced hydrocarbon compound, and are excellent in the effect of enhancing the yield of the Guerbet alcohol compound formed, as compared to Comparative Example 1 using the catalyst a1.

### (Summary of Results 4)

### Table 4: Investigation into Raw Material Alcohol

It is found that Example 9 using 1-dodecanol (C12) as the raw material alcohol is excellent in the effect of enhancing the yield of the Guerbet alcohol compound formed as compared to Example 10 using 1-hexadecanol (C16) as the raw material alcohol.

## Claims

1. A method of producing a Guerbet alcohol, comprising reacting a raw material alcohol having 8 or more and 36 or less carbon atoms, in the presence of a catalyst (A) containing a first component and a second component below, having a molar ratio of the first component with respect to the second component (first component/second component) of 2.9 or less:
first component: copper, and
second component: one kind selected from the group consisting of elements that are elements belonging to Groups 3 to 12 in the fourth to sixth periods of the periodic table, except copper and nickel.

2. The method of producing a Guerbet alcohol according to claim 1, wherein the second component of the catalyst (A) is an element belonging to the fourth period of the periodic table.

3. The method of producing a Guerbet alcohol according to claim 1 or 2, wherein the second component of the catalyst (A) is zinc.

4. The method of producing a Guerbet alcohol according to any one of claims 1 to 3, wherein the catalyst (A) has a molar ratio of the first component with respect to the second component (first component/second component) of 0.11 or more.

5. The method of producing a Guerbet alcohol according to any one of claims 1 to 4, wherein the catalyst (A) is a catalyst (A) including a carrier having the first component and the second component supported thereon.

6. The method of producing a Guerbet alcohol according to claim 5, wherein the carrier of the catalyst (A) is at least one kind selected from the group consisting of zeolite, hydrotalcite, aluminum oxide, activated carbon, titanium oxide, zirconium oxide, and cerium oxide.

7. The method of producing a Guerbet alcohol according to any one of claims 1 to 6, wherein the catalyst (A) has a content of the first component contained therein of 4% by mass or more and 50% by mass or less.

8. The method of producing a Guerbet alcohol according to any one of claims 1 to 7, wherein in suspended bed reaction, an amount of the catalyst (A) per 100 parts by mass in total of the amount of the raw material alcohol is 0.001 part by mass or more and 10 parts by mass or less.

9. The method of producing a Guerbet alcohol according to any one of claims 1 to 8, wherein a base catalyst (B) is used with the catalyst (A).

10. The method of producing a Guerbet alcohol according to claim 9, wherein an amount of the base catalyst (B) per 100 parts by mol in total of the amount of the raw material alcohol is 0.1 part by mol or more and 7 parts by mol or less.

11. The method of producing a Guerbet alcohol according to any one of claims 1 to 10, wherein the raw material alcohol is a primary aliphatic alcohol having 8 or more and 18 or less carbon atoms.

12. A catalyst used for a method of producing a Guerbet alcohol, comprising a first component and a second component below, having a molar ratio of the first component with respect to the second component (first component/second component) of 2.9 or less:
first component: copper, and
second component: one kind selected from the group consisting of elements that are elements belonging to Groups 3 to 12 in the fourth to sixth periods of the periodic table, except copper and nickel.
